# EUROPÄISCHE PATENTSCHRIFT

(11) **EP 0 937 734 B1**
(45) Veröffentlichungstag und Bekanntmachung des Hinweises auf die Patenterteilung: **27.10.2010**
(21) Anmeldenummer: 99101675.9
(22) Anmeldetag: 08.02.1999
(51) Int. Cl.: C07H 17/08, A61K 31/7048

(54) **11-Acetyl-12,13-dioxabicyclo ¬8.2.1 tridecenon-Derivate, Verfahren zu ihrer Herstellung und diese Verbindungen enthaltende Arzneimittel**
11-Acetyl-12,13-dioxabicyclo ¬8.2.1 tridecenone derivatives, process for making them and pharmaceutical compositions containing them
Dérivés de la 11-acétyl-12,13-dioxabicyclo ¬8.2.1 tridécénone, procédé de fabrication et compositions pharmaceutiques les contenant

(30) Priorität: 13.02.1998 DE 19805822
(43) Veröffentlichungstag der Anmeldung: 25.08.1999
(73) Patentinhaber: Abbott Products GmbH, 30173 Hannover (DE)
(72) Erfinder: Jasserand, Daniel, Dr., 30625 Hannover (DE); Preuschoff, Ulf, Dr., 29125 Uelzen (DE); Eeckhout, Christian, Dr., 29690 Lindwedel (DE)
(74) Vertreter: Gosmann, Martin

(56) Entgegenhaltungen:
- EP-A- 0 349 100
- EP-A- 0 382 472
- EP-A- 0 550 895

## Beschreibung

Die vorliegende Erfindung betrifft neue N-substituierte (2R,3S,4S,5R,6R,10R,11R)-3-[(2,6-Didesoxy-3-C-methyl-3-O-methyl-α-L-ribohexopyranosyl)-oxy]-5-[(3,4,6-tridesoxy-3-amino-β-D-xylohexopyranosyl)-oxy]-2,4,6,8,10-pentamethyl-11-acetyl-12,13-dioxabicyclo[8.2.1]tridec-8-en-1-on-Verbindungen mit Motilin-agonistischen Eigenschaften und deren Säureadditionssalze sowie diese Verbindungen enthaltende pharmazeutische Zubereitungen und Verfahren zur Herstellung dieser Verbindungen. Die erfindungsgemäßen Verbindungen stellen ringverengte N-Desmethyl-N-isopropyl-Derivate des Erythromycin A mit modifizierter Seitenkette dar.

Das Antibiotikum Erythromycin A besitzt bekanntermaßen neben seinen antibiotischen Wirkungen auch für Antibiotika unerwünschte gastrointestinale Nebenwirkungen, unter anderem eine starke Vermehrung der Kontraktionsaktivität im Magen-Darm-Bereich mit Magen- und Darmkrämpfen, Übelkeit, Erbrechen und Diarrhöe.

Es hat mehrere Versuche gegeben, das Erythromycin A so abzuwandeln, daß Derivate erhalten werden, in denen die antibiotische Wirkung praktisch nicht mehr vorhanden ist, jedoch eine die Motilität des gastrointestinalen Traktes beeinflussende Wirkung erhalten ist. Aus dem europäischen Patent Nr. 0 550 895 sind ringverengte N-Desmethyl-N-isopropyl-erythromycin-A-Derivate mit gastrointestinal wirksamen Motilin-agonistischen Eigenschaften bekannt.

Weiterhin sind aus der europäischen Patentanmeldung EP-A 382 472 ähnliche ringverengte Erythromycinderivate bekannt, welche aber antibiotische Wirkungen zeigen.

Der vorliegenden Erfindung liegt die Aufgabe zugrunde, neue oral wirksame ringverengte Derivate des Erythromycin A ohne Antibiotikawirkung und mit die Motilität des gastrointestinalen Traktes günstig beeinflussenden Eigenschaften mit einem verbessertem Wirkprofil zu entwickeln.

Es wurde nun gefunden, daß die neuen ringverengten, N-Desmethyl-N-isopropyl-derivate des Erythromycin A, deren Seitenkette in 11-Position des cyclischen Grundgerüstes durch Oxidation modifiziert wurde, nicht antibiotisch wirksam sind, aber selektive Motilin-agonistische Eigenschaften aufweisen und die Motilität des gastrointestinalen Traktes in günstiger Weise stimulieren und den Tonus des unteren Oesophagus Sphincter und den Tonus des Magens verstärkende Wirkungen zeigen.

Aufgrund ihres Wirkungsprofils eignen sich die erfindungsgemäßen Substanzen zur Behandlung von Motilitätsstörungen im gastrointestinalen Trakt und zeichnen sich dabei durch eine gute Verträglichkeit und gute orale Wirksamkeit aus.

Die vorliegende Erfindung betrifft daher neue (2R,3S,4S,5R,6R,10R,11R)-2,4,6,8,10-Pentamethyl-11-acetyl-12,13-dioxabicyclo[8.2.1]tridec-8-en-1-on-Verbindungen der allgemeinen Formel I 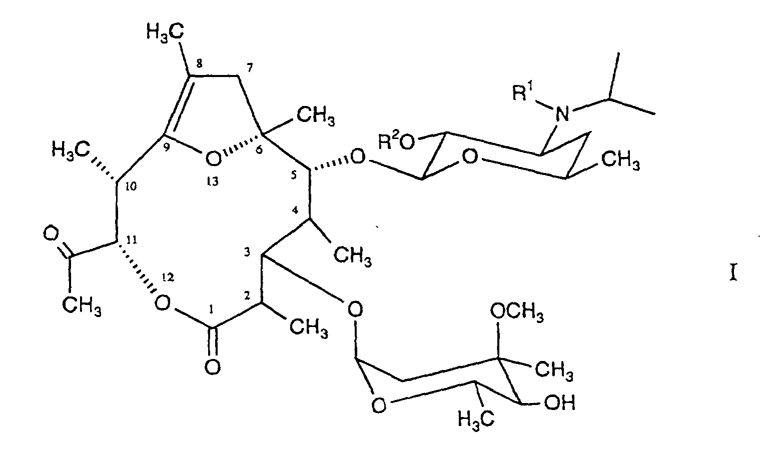 worin
- R¹: Wasserstoff oder Methyl bedeutet und
- R²: Wasserstoff oder niederes Alkanoyl bedeutet
und deren stabile und physiologisch verträgliche Säureadditionssalze.

Sofern in Verbindungen der Formel I ein Substituent niederes Alkyl bedeutet oder enthält, kann dieses verzweigt oder unverzweigt sein und 1 bis 4 Kohlenstoffatome besitzen.

Als günstig erweisen sich insbesondere die Verbindungen der Formel I, worin R¹ Methyl bedeutet.

R² steht bevorzugt für Wasserstoff. Sofern R² niederes Alkanoyl bedeutet, ist Acetyl bevorzugt.

Die Verbindungen der Formel I können erhalten werden, indem man auf an sich bekannte Weise in einer Verbindung der allgemeinen Formel II, 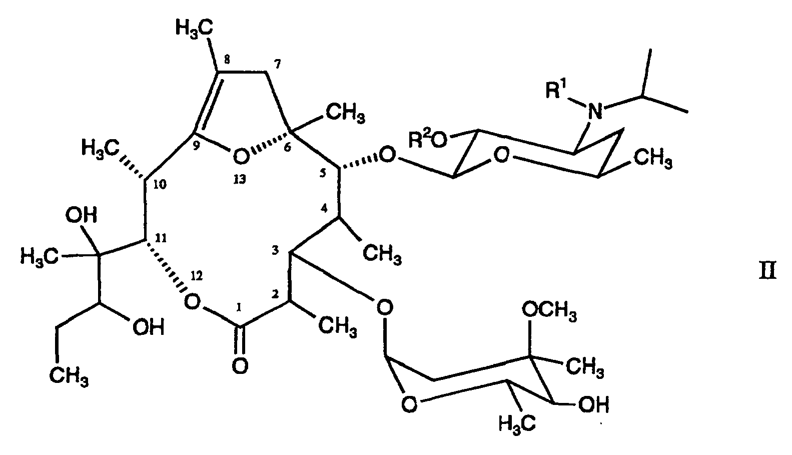 worin R¹ und R² obige Bedeutungen besitzen, die 2',3'-Dihydroxypent-2'-yl-Seitenkette in 11-Position des cyclischen Grundgerüstes durch oxidative Glykolspaltung in eine Acetyl-Seitenkette überführt und gewünschtenfalls in die erhaltene Verbindung der Formel I, worin R¹ Wasserstoff bedeutet, einen Methylrest R¹ einführt oder in der erhaltenen Verbindung der Formel I, worin R¹ Methyl bedeutet, den Methylrest R¹ abspaltet und gewünschtenfalls freie Verbindungen der Formel I in ihre stabilen und physiologisch verträglichen Säureadditionssalze überführt oder die Säureadditionssalze in die freien Verbindungen der Formel I überführt.

Die oxidative Glykolspaltung der 2',3'-Dihydroxypent-2'-yl-Seitenkette in 11-Position des cyclischen Grundgerüstes von Verbindungen der Formel II kann mit geeigneten Oxidationsmitteln wie Bleitetraacetat in hierfür geeigneten Lösungsmitteln durchgeführt werden. Als Lösungsmittel eignen sich unpolare oder schwachpolare Lösungsmittel wie Benzol, Toluol oder Xylol. Die Reaktion kann bei Temperaturen zwischen 0 °C und 40 °C, bevorzugt bei Raumtemperatur, ausgeführt werden.

Die erhaltenen Verbindungen der Formel I, worin R¹ Wasserstoff bedeutet, können gewünschtenfalls nachträglich in an sich bekannter Weise zu den entsprechenden N-Methyl-Verbindungen alkyliert werden. Die Alkylierung kann auf an sich bekannte Weise durch Umsetzen mit einem Methylhalogenid oder als reduktive Alkylierung durch Umsetzen mit Formaldehyd unter reduzierenden Bedingungen erfolgen und kann beispielsweise in Gegenwart eines Reduktionsmittels, beispielsweise einer komplexen Borhydridverbindung wie Natriumcyanoborhydrid, Natriumtriacetoxyborhydrid oder Natriumborhydrid, durchgeführt werden. Gewünschtenfalls kann die Alkylierung auch durch Umsetzen mit einem Methylhalogenid, insbesondere Methyljodid oder einem Methylsulfonsäureester erfolgen. Zweckmäßigerweise wird die Alkylierung in einem unter den Reaktionsbedingungen inerten organischen Lösungsmittel durchgeführt. Für die reduktive Alkylierung eignen sich als Lösungsmittel cyclische Ether wie Tetrahydrofuran (= THF) oder Dioxan, aromatische Kohlenwasserstoffe wie Toluol oder auch niedere Alkohole. Die Alkylierung kann bei Temperaturen zwischen Raumtemperatur und Siedetemperatur des Lösungsmittels erfolgen. Bei der Alkylierung mit einem Methylderivat, beispielsweise einem Methylhalogenid wie Methyljodid, wird zweckmäßigerweise in Gegenwart einer Base wie beispielsweise einem Alkalimetallcarbonat oder einem tertiären organischen Amin gearbeitet.

Aus den Verbindungen der Formel I, worin R¹ Methyl bedeutet, kann der Methylrest R¹ gewünschtenfalls nachträglich abgespalten werden. Die Demethylierung kann auf an sich bekannte Weise durch Behandeln der Verbindung mit einem Halogen, insbesondere Jod und/oder Brom, in einem inerten Lösungsmittel in Gegenwart einer geeigneten Base erfolgen. Als Basen eignen sich beispielsweise Alkalimetallalkoholate, Alkalimetallhydroxide und Alkalimetallsalze von schwachen organischen Säuren.

Die Verbindungen der Formel I können auf an sich bekannte Weise aus dem Reaktionsgemisch isoliert und gereinigt werden. Säureadditionssalze können in üblicher Weise in die freien Basen überführt werden und diese können gewünschtenfalls in bekannter Weise in pharmakologisch verträgliche Säureadditionssalze überführt werden. Zur Vermeidung von Hydrolysenebenreaktionen ist es zweckmäßig, zur Salzbildung nur äquivalente Mengen Säuren zu verwenden.

Als pharmakologisch annehmbare Säureadditionssalze der Verbindungen der Formel I eignen sich beispielsweise deren Salze mit anorganischen Säuren, z. B. Kohlensäure, Halogenwasserstoffsäuren, insbesondere Chlorwasserstoffsäure, oder mit organischen Säuren, beispielsweise niederen aliphatischen Mono- oder Dicarbonsäuren wie Maleinsäure, Fumarsäure, Milchsäure, Weinsäure oder Essigsäure.

Die Ausgangsverbindungen der Formel II, worin R² Wasserstoff bedeutet, sind aus der EP-B 0 550 895 bekannt und können nach den dort beschriebenen Verfahren hergestellt werden.

Die Ausgangsverbindungen der Formel II, worin R² niederes Alkanoyl bedeutet, können hergestellt werden, indem man Verbindungen der Formel II, worin R² Wasserstoff bedeutet, mit Carbonsäuren der allgemeinen Formel III,

R³-COOH III

worin R³ niederes Alkyl bedeutet, oder reaktionsfähigen Derivaten dieser Säuren, auf an sich bekannte Weise umsetzt.

Als reaktionsfähige Derivate von Säuren der Formel III kommen insbesondere gegebenenfalls gemischte Säureanhydride und Säurehalogenide in Frage. So können beispielsweise Säurechloride oder Säurebromide der Säuren der Formel III oder gemischte Ester der Säuren der Formel III mit organischen Sulfonsäuren, beispielsweise mit gegebenenfalls durch Halogen substituierten Niederalkansulfonsäuren wie Methansulfonsäure oder Trifluormethansulfonsäure oder mit aromatischen Sulfonsäuren wie z. B. Benzolsulfonsäuren oder mit durch niederes Alkyl oder Halogen substituierten Benzolsulfonsäuren, z. B. Toluolsulfonsäuren oder Brombenzolsulfonsäuren, eingesetzt werden. Die Umsetzung kann als Acylierung in einem unter den Reaktionsbedingungen inerten organischen Lösungsmittel bei Temperaturen zwischen -20 °C und Raumtemperatur erfolgen. Als Lösungsmittel eignen sich Diniederalkylketone, beispielsweise Aceton, halogenierte Kohlenwasserstoffe wie Dichlormethan oder aromatische Kohlenwasserstoffe wie Benzol oder Toluol oder cyclische Ether wie THF oder Dioxan oder Gemische dieser Lösungsmittel.

Die Acylierung kann zweckmäßig, insbesondere wenn als Acylierungsmittel ein Anhydrid oder ein gemischtes Anhydrid der Säuren der Formel III mit einer Sulfonsäure verwendet wird, in Gegenwart eines säurebindenden Reagenzes durchgeführt werden. Als säurebindende Mittel eignen sich beispielsweise anorganische Basen wie Alkalimetallcarbonate, beispielsweise Kaliumcarbonat, oder in dem Reaktionsgemisch lösliche organische Basen wie tertiäre Stickstoffbasen, beispielsweise tert.-Niederalkylamine und Pyridine wie z. B. Triethylamin, Tripropylamin, N-Methylmorpholin, Pyridin, 4-Dimethylaminopyridin, 4-Diethylaminopyridin oder 4-Pyrrolidinopyridin.

Gewünschtenfalls kann in eine erhaltene Verbindung der Formel II, worin R¹ Wasserstoff bedeutet, ein Methylrest R¹ eingeführt oder in einer erhaltenen Verbindung der Formel II, worin R¹ Methyl bedeutet, der Methylrest R¹ abgespalten werden. Derartige Methylierungen oder Demethylierungen können auf an sich bekannte Weise, beispielsweise unter den für die Einführung oder Abspaltung einer Methylgruppe in den Verbindungen der Formel I beschriebenen Bedingungen erfolgen.

Die neuen Verbindungen der Formel I und ihre stabilen und physiologisch verträglichen Säureadditionssalze besitzen interessante pharmakologische Eigenschaften, insbesondere die Motilität des gastrointestinalen Traktes stimulierende Motilin-agonistische Eigenschaften. Dabei zeichnen sie sich durch ein günstiges Wirkprofil mit überraschend guter oraler Wirksamkeit aus. Sie sind frei von antibiotischen Wirkungen und besitzen eine hohe selektive Affinität zu Motilin-Rezeptoren, während sie in Motilin-agonistisch wirksamen Dosisbereichen keine praktisch relevante Affinität zu anderen Rezeptoren im gastrointestinalen Trakt wie Adrenalin-, Acetylcholin-, Histamin-, Dopamin- oder Serotonin-Rezeptoren zeigen. Die Verbindungen weisen eine überraschend gute Leberverträglichkeit auf, welche sie für Anwendungen über längere Zeiträume hin geeignet macht.

Um eine geregelte Verdauung der eingenommenen Nahrung zu gewährleisten, wirken im gesunden Zustand das autonome Nervensystem und Hormone des gastrointestinalen Traktes zusammen, um eine geregelte Kontraktionstätigkeit des gastrointestinalen Traktes nicht nur direkt nach Nahrungsaufnahme, sondern auch bei leerem gastrointestinalen Trakt zu erzeugen. Motilin ist ein bekanntes gastrointestinales Peptidhormon, welches die Motilität des gastrointestinalen Traktes stimuliert und eine koordinierte Motilität im gesamten gastrointestinalen Trakt im nüchternen Zustand sowie nach Nahrungsaufnahme induziert.

Die Verbindungen der Formel I zeigen Motilin-artige physiologische Wirkungen, indem sie als Agonisten für Motilinrezeptoren wirksam werden. So zeigen die Verbindungen der Formel I ausgeprägte stimulierende Wirkungen im Magen-Darm-Bereich und am unteren Speiseröhrensphincter. Sie bewirken insbesondere eine Beschleunigung der Magenentleerung, eine Erhöhung des Magentonus und eine langanhaltende Erhöhung des Ruhe-Tonus des Oesophagus Sphincter. Aufgrund ihres Motilinartigen Wirkungsprofils eignen sich die Substanzen zur Behandlung von Krankheitszuständen, welche mit Motilitätsstörungen im gastrointestinalen Trakt und/oder Rückfluß von Speisebrei aus dem Magen in die Speiseröhre verbunden sind. So sind die Verbindungen der Formel I z. B. indiziert bei Gastroparesis verschiedensten Ursprungs, Störungen des Magentonus, Störungen der Magenentleerung und gastroösophagalem Rückfluß, Dyspepsie und postoperativen Motilitätsstörungen.

Die gastrointestinal wirksamen Eigenschaften der Verbindungen der Formel I lassen sich in pharmakologischen Standardtestmethoden in vitro und in vivo nachweisen.

### Beschreibung der Testmethoden

### 1. Bestimmung des Bindungsvermögens der Testsubstanzen an Motilin-Rezeptoren.

Die Affinität der Verbindungen der Formel I an Motilin-Rezeptoren wird in vitro an einer Fraktion eines Gewebehomogenats aus dem Kaninchenantrum gemessen. Bestimmt wird die Verdrängung von radioaktiv markiertem jodiertem Motilin aus der Motilin-Rezeptor-Bindung durch die Testsubstanzen.

Die Rezeptor-Bindungsstudien werden nach einer Modifikation der Methode von Borman et al. (Regulatory Peptides 15 (1986), 143-153) durchgeführt. Zur Herstellung des ¹²⁵Jodmarkierten Motilins wird Motilin auf an sich bekannte Weise, z. B. analog der von Bloom et al. (Scand. J. Gastroenterol. 11 (1976) 47 - 52) beschriebenen Methode enzymatisch unter Verwendung von Lactoperoxidase jodiert.

Zur Gewinnung der in dem Test verwendeten Gewebehomogenatfraktion aus dem Kaninchenantrum wird das von Schleimhäuten befreite Antrum zerkleinert und im 10-fachen Volumen einer kalten Homogenisierungspufferlösung (50 mM Tris-HCl-Puffer, 250 mM Sucrose, 25 mM KCl, 10 mM MgCl₂, pH 7,4) mit Zusatz von Inhibitoren (1 mM Jodacetamid, 1 µM Pepstatin, 0,1 mM Methylsulfonylfluorid, 0,1 g/l Trypsininhibitor, 0,25 g/l Bacitracin) mit einem Homogenisator 15 sec. bei 1500 Umdrehungen pro Minute homogenisiert. Das Homogenisat wird dann 15 Minuten lang bei 1000 g zentrifugiert, der erhaltene Rückstand wird viermal mit Homogenisierungspufferlösung gewaschen und schließlich in 0,9%iger Natriumchloridlösung (in einem der 5-fachen Gewichtsmenge des Antrums entsprechendem Volumen) resuspendiert. Die so erhaltene Gewebefraktion, welche als "rohe Membranzubereitung" bezeichnet wird, wird für den Test eingesetzt.

Für den Bindungsversuch werden 200 µl der rohen Membranfraktion (0,5 - 1 mg Protein) in 400 µl einer Pufferlösung A (50 mM Tris-HCl-Puffer, 1,5 % Rinderserumalbumin (= Bovine Serum Albumin, BSA), 10 mM MgCl₂, pH 8,0) mit 100 µl jodiertem Motilin in Pufferlösung B (10 mM Tris-HCl-Puffer, 1 % BSA, pH 8,0) verdünnt (Endkonzentration 50 pM) 60 min. bei 30 °C inkubiert. Die Reaktion wird durch Zugabe von 3,2 ml kalter Pufferlösung B gestoppt und gebundenes und nichtgebundenes Motilin werden durch Zentrifugieren (1000 g, 15 Minuten) voneinander getrennt. Der nach dem Zentrifugieren als Pellet erhaltene Rückstand wird mit Pufferlösung B gewaschen und in einem Gamma-Zähler ausgezählt. Die Verdrängungsstudien werden durch Zugabe steigender Mengen der zu testenden Substanz in das Inkubationsmedium durchgeführt. Als Testsubstanzlösungen werden wäßrige Lösungen eingesetzt, welche durch geeignete Verdünnung von 60 x 10⁻⁴-molaren wäßrigen Stammlösungen hergestellt werden. In Wasser schwer lösliche Testsubstanzen werden zunächst in 60%igem Ethanol gelöst und diese Lösung wird mit soviel Wasser verdünnt, daß in der zu testenden Lösung die Ethanolkonzentration 1,6 Vol.-% nicht übersteigt. Aus den erhaltenen Meßdaten wird als IC₅₀ der jeweiligen Testsubstanz diejenige Konzentration bestimmt, welche eine 50%ige Hemmung der spezifischen Bindung des jodierten Motilin an die Motilin-Rezeptoren bewirkt. Aus dieser wird der entsprechende pIC₅₀-Wert berechnet. Nach der vorstehenden Methode wurden für die Substanzen der Beispiele 1 und 2 die in der nachfolgenden Tabelle 1 angegebenen pIC₅₀-Werte bestimmt. Die angegebenen Beispielsnummern beziehen sich auf die nachfolgend beschriebenen Herstellungsbeispiele.

**Tabelle 1**

| **Beispiel Nr.** | **pIC₅₀** |
|---|---|
| 1 | 8,01 |
| 2 | 7,75 |

### 2. In-vivo-Bestimmung des Einflusses der Substanzen auf den Magentonus

Der Magentonus spielt eine wichtige Rolle bei der Magenentleerung. Ein erhöhter Magentonus trägt zu einer beschleunigten Magenentleerung bei.

Der Einfluß von Substanzen auf den Magentonus wird an Beagle-Hunden mit Hilfe eines Barostaten bestimmt, welcher mit einem Kunststoffbeutel im Magen des Hundes verbunden ist und die Messung von Volumen oder Druck im Magen des Hundes ermöglicht. Mit dem Barostaten wird das Magenvolumen bei konstantem Druck im Magen oder der Magendruck bei konstantem Volumen im Magen bestimmt. Bei Erhöhung des Magentonus stellt man bei einem bestimmten Druck ein verringertes Magenvolumen fest und einen erhöhten Druck bei einem bestimmten Volumen. In dem zur Untersuchung der durch die Substanzen bewirkten Erhöhung des Magentonus verwendeten Testmodell wird die durch die Substanzen verursachte Magenvolumenänderung bei konstantem Druck gemessen. Der Magen der Versuchstiere wird durch Lipideinnahme relaxiert, d. h. der Magentonus sinkt, wodurch das Magenvolumen entsprechend zunimmt. Als Maß für die den Magentonus erhöhende Wirkung der Substanzen wird die nach Substanzeinnahme durch Wiederanstieg des Magentonus eintretende Reduktion des durch Lipidgabe vergrößerten Magenvolumens in % gemessen.

Die Substanz des Beispieles 1 zeigte in diesem Testmodell, in der gut verträglichen Dosis von 2,15 µmol/kg i.d. verabreicht, eine Reduktion des nach Lipidgabe vergrößerten Magenvolumens um 59,5 %. Die orale Verabreichung der vorstehend angegebenen Testsubstanz in derselben Dosis von 2,15 µmol/kg bewirkte eine ungewöhnlich ausgeprägte Reduktion des Magenvolumens, wobei die Lipid-induzierte Relaxierung des Magenvolumens praktisch völlig verhindert wurde. Diese Befunde können als deutliche Indizien für eine besonders hohe, insbesondere hohe orale, Bioverfügbarkeit der erfindungsgemäßen Substanzen gewertet werden.

### 3. In-vivo-Bestimmung des Einflusses der Substanzen auf den Ruhe-Tonus des unteren Oesophagus Sphincter.

Diese Bestimmung wird an männlichen, wachen, nüchternen Beagle-Hunden vorgenommen, denen vor Versuchsbeginn je eine Oesophagus-Fistel und eine Duodenal-Kanüle gelegt worden sind. Der Druck des unteren Oesophagus Sphincter wird mittels eines perfundierten Kathetersystems mit seitlicher Öffnung, das mit einem Druckaufnehmer und einem Recorder verbunden ist, gemessen. Der Katheter wird über die Oesophagus-Fistel in den Magen geführt und dann langsam manuell zurückgezogen (= Durchzugsmanometrie). Beim Passieren des Katheterteils mit der seitlichen Öffnung durch die Hochdruckzone des unteren Oesophagus Sphincter wird ein Peak registriert. Aus diesem Peak wird der Druck in mm Hg bestimmt.

Auf diese Weise wird zunächst als Kontrollwert der basale Druck des Oesophagus Sphincter bestimmt. Anschließend wird die Testsubstanz oral appliziert und nach 15 min. wird der Druck am unteren Oesophagus Sphincter in 2-Minuten-Intervallen über einen Zeitraum von 60 min. gemessen. Die Steigerung des Druckes nach Testsubstanzgabe im Vergleich zu dem vorbestimmten basalen Druck wird berechnet.

In diesem Test nahm der basale Tonus des Oesophagus Sphincter durch eine Dosis von 2,15 µMol/kg der Substanz des Beispiels 1 um 143 % zu. Dieser Effekt hielt während der gesamten Testdauer von 60 min. an.

Aufgrund ihrer Wirkungen im gastrointestinalen Trakt sind die Verbindungen der Formel I in der Gastroenterologie als Arzneimittel für größere Säugetiere, insbesondere Menschen, zur Prophylaxe und Behandlung von Motilitätsstörungen des gastrointestinalen Traktes geeignet.

Die zu verwendenden Dosen können individuell verschieden sein und variieren naturgemäß je nach Art des zu behandelnden Zustandes und der Applikationsform. Zum Beispiel werden parenterale Formulierungen im allgemeinen weniger Wirkstoff enthalten als orale Präparate. Im allgemeinen eignen sich jedoch für Applikationen an größeren Säugetieren, insbesondere Menschen, Arzneiformen mit einem Wirkstoffgehalt von 1 bis 100 mg pro Einzeldosis.

Als Heilmittel können die Verbindungen der Formel I mit üblichen pharmazeutischen Hilfsstoffen in galenischen Zubereitungen wie z. B. Tabletten, Kapseln, Suppositorien oder Lösungen enthalten sein. Diese galenischen Zubereitungen können nach an sich bekannten Methoden hergestellt werden unter Verwendung üblicher fester Trägerstoffe, wie z. B. Milchzukker, Stärke oder Talkum oder flüssiger Verdünnungsmittel, wie z. B. Wasser, fetten Ölen oder flüssigen Paraffinen und unter Verwendung von pharmazeutisch üblichen Hilfsstoffen, beispielsweise Tablettensprengmitteln, Lösungsvermittlern oder Konservierungsmitteln.

Die nachfolgenden Beispiele sollen die Erfindung näher erläutern, jedoch deren Umfang in keiner Weise beschränken.

### Beispiel 1:

(2R,3S,4S,5R,6R,10R,11R]-3-[(2,6-Didesoxy-3-C-methyl-3-O-methyl-α-L-ribohexopyranosyl)-oxy]-5-[(3,4,6-tridesoxy-3-(N-methyl-N-isopropylamino)-β-D-xylohexopyranosyl)-oxy]-2,4,6,8,10-pentamethyl-11-acetyl-12,13-dioxabicyclo[8.2.1]-tridec-8-en-1-on (Verbindung der Formel I, R¹ = Methyl,
R² = Wasserstoff)
A) 100 g [2R(2'R,3'R),3S,4S,5R,6R,10R,11R]-11-(2',3'-Dihydroxypent-2'-yl)-3-[(2,6-didesoxy-3-C-methyl-3-O-methyl-α-L-ribohexopyranosyl)-oxy]-5-[(3,4,6-tridesoxy-3-(N-methyl-N-isopropylamino)-β-D-xylohexopyranosyl)-oxy]-2,4,6,8,10-pentamethyl-12,13-dioxabicyclo[8.2.1]tridec-8-en-1-on (= Verbindung der Formel II, R¹ = Methyl, R² = Wasserstoff) wurden unter Stickstoffatmosphäre in 2500 ml Toluol gelöst. Zu dieser Vorlage gab man 100,0 g Bleitetraacetat und rührte die entstandene Suspension 5 Stunden lang bei Raumtemperatur. Anschließend wurde die Reaktionsmischung der Reihe nach mit gesättigter Natriumhydrogencarbonatlösung und dann so oft mit Wasser gewaschen, bis das Waschwasser neutral reagierte. Die organische Phase wurde über Natriumsulfat getrocknet und unter vermindertem Druck eingedampft. Chromatographie des verbliebenen Rückstandes an Kieselgel (Laufmittel: Methyl-tert.-butylether = MTBE) lieferte 81,9 g der Titelverbindung als weißes Pulver, Fp. = 198° - 200 °C, optischer Drehwert [α]_{D}²⁰ = -24,6° (c = 1,0 in CH₂Cl₂).
B) 1,1 g der vorstehend erhaltenen Verbindung wurden in 1 ml Acetonitril gelöst. Zu dieser Vorlage gab man 0,17 g Malonsäure und erwärmte auf 60° - 70 °C. Nach Auflösung der festen Bestandteile wurden 10 ml MTBE zugegeben und es wurde 5 Minuten lang unter Rückflußkühlung zum Sieden erhitzt. Anschließend gab man erneut 10 ml MTBE zu und ließ das Gemisch unter Rühren auf Raumtemperatur abkühlen. Die ausgefallenen Kristalle wurden von der Lösung abfiltriert, zweimal mit je 10 ml MTBE gewaschen und bei 60 °C im Vakuum getrocknet. Man erhielt 1,2 g des Monomalonates der Titelverbindung, Schmelzbereich: 115,6 - 174,6 °C (unscharf).

### Beispiel 2:

(2R,3S,4S,5R,6R,10R,11R)-3-[(2,6-Didesoxy-3-C-methyl-3-O-methyl-α-L-ribohexopyranosyl)-oxy]-5-[(3,4,6-tridesoxy-2-O-acetyl-3-(N-methyl-N-isopropylamino)-β-D-xylohexopyranosyl)-oxy]-2,4,6,8,10-pentamethyl-11-acetyl-12,13-dioxabicyclo-[8.2.1]tridec-8-en-1-on (Verbindung der Formel I,
R¹ = Methyl, R² = Acetyl)
A) 210,0 g der Ausgangsverbindung aus Beispiel 1 (= Verbindung der Formel II, R¹ = Methyl, R² = Wasserstoff) wurden unter Stickstoffatmosphäre in 2,4 l Aceton gelöst und mit 85,8 g Kaliumcarbonat versetzt. Zu dieser Vorlage wurden 63,4 g Essigsäureanhydrid hinzugefügt und die erhaltene Suspension wurde 20 Stunden lang bei Raumtemperatur gerührt. Anschließend wurde das Reaktionsgemisch auf eine Mischung aus 2.400 g Eis und 1.000 ml Wasser gegossen und 30 Minuten lang gerührt. Die wäßrige Phase wurde dreimal mit Essigsäureethylester extrahiert, die organischen Phasen vereinigt und das überschüssige Lösungsmittel wurde im Vakuum eingedampft. Umkristallisation des erhaltenen Rohproduktes aus n-Pentan lieferte 200 g [2R(2'R,3'R),3S,4S, 5R,6R,10R,11R]-11-(2',3'-Dihydroxypent-2'-yl)-3-[(2,6-didesoxy-3-C-methyl-3-O-methyl-α-L-ribohexopyranosyl)-oxy]-5-[(3,4,6-tridesoxy-2-O-acetyl-3-(N-methyl-N-isopropylamino)-β-D-xylohexopyranosyl)-oxy]-2,4,6,8,10-pentamethyl-12,13-dioxabicyclo[8.2.1]tridec-8-en-1-on (= Verbindung der Formel II, R¹ = Methyl, R² = Acetyl) Fp. = 128° - 130 °C.
B) 10,1 g des vorstehend erhaltenen Produktes wurden auf die in Beispiel 1 beschriebene Weise mit 9,1 g Bleitetraacetat umgesetzt. Man erhielt 6,0 g der Titelverbindung als weiβen Feststoff, Fp. = 164 °C optischer Drehwert [α]_{D}²⁰ = -23,2° (c = 1,0 in CH₂Cl₂).

### Beispiel I:

(2R,3S,4S,5R,6R,10R,11R)-3-[(2,6-Didesoxy-3-C-methyl-3-O-methyl-α-L-ribohexopyranosyl)-oxy]-5-[(3,4,6-tridesoxy-3-(N-methyl-N-isopropylamino)-β-D-xylohexopyranosyl)-oxy]-2,4,6,8,10-pentamethyl-11-acetyl-12,13-dioxabicyclo[8.2.1]-tridec-8-en-1-on enthaltende Kapseln:

Man stellte den Wirkstoff enthaltende Kapseln unter Verwendung der folgenden Hilfs- und Inhaltsstoffe pro Kapsel her:

| | |
|---|---|
| (2R,3S,4S,5R,6R,10R,11R)-3-[(2,6-Didesoxy-3-C-methyl-3-O-methyl-α-L-ribohexopyranosyl)-oxy]-5-[(3,4,6-tridesoxy-3-(N-methyl-N-isopropylamino)-β-D-xylohexopyranosyl)-oxy]-2,4,6,8,10-pentamethyl-11-acetyl-12,13-dioxabicyclo[8.2.1]tridec-8-en-1-on | 20 mg |
| Maisstärke | 60 mg |
| Milchzucker | 301 mg |
| Essigsäureethylester (= EE) | q.s. |

Der Wirkstoff, die Maisstärke und der Milchzucker wurden unter Zuhilfenahme von EE zu einer homogenen pastösen Mischung verarbeitet. Die Paste wurde zerkleinert und das entstehende Granulat wurde auf ein geeignetes Blech gebracht und zur Entfernung des Lösungsmittels bei 45 °C getrocknet. Das getrocknete Granulat wurde durch eine Zerkleinerungsmaschine geleitet und in einem Mixer mit weiteren folgenden Hilfsstoffen vermischt:

| | |
|---|---|
| Talkum | 5 mg |
| Magnesiumstearat | 5 mg |
| Maisstärke | 9 mg |

und sodann in 400 mg fassende Kapseln (= Kapselgröße 0) abgefüllt.

## Patentansprüche

1. (2R,3S,4S,5R,6R,10R,11R)-2,4,6,8,10-Pentamethyl-11-acetyl-12,13-dioxabicyclo[8.2.1]tridec-8-en-1-on-Verbindungen der allgemeinen Formel I 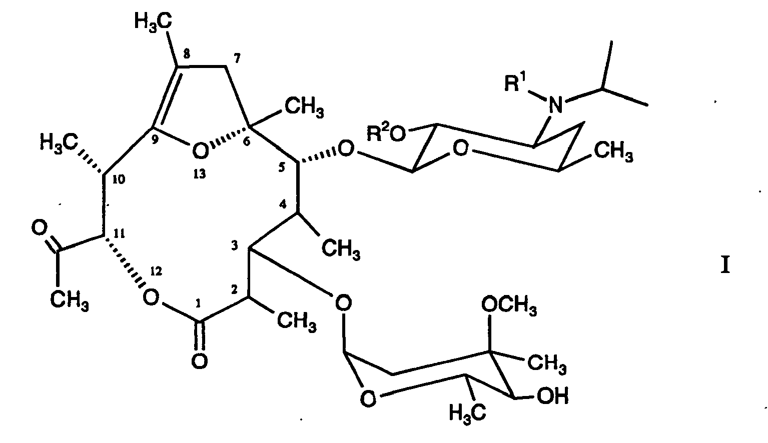 worin
R¹ Wasserstoff oder Methyl bedeutet und
R² Wasserstoff oder C₁₋₄-Alkylcarbonyl bedeutet
und deren stabile und physiologisch verträgliche Säureadditionssalze.

2. Verbindungen gemäß Anspruch 1, worin R¹ Methyl bedeutet.

3. Verbindungen gemäß einem der vorstehenden Ansprüche, worin R² Wasserstoff bedeutet.

4. Arzneimittel, enthaltend eine pharmakologisch wirksame Menge einer Verbindung gemäß Anspruch 1 und übliche pharmazeutische Hilfs- und/oder Trägerstoffe.

5. Verfahren zur Herstellung von (2R,3S,4S,5R,6R,10R,11R)-2,4,6,8,10-Pentamethyl-11-acetyl-12,13-dioxabicyclo[8.2.1]-tridec-8-en-1-on-Verbindungen der allgemeinen Formel I, 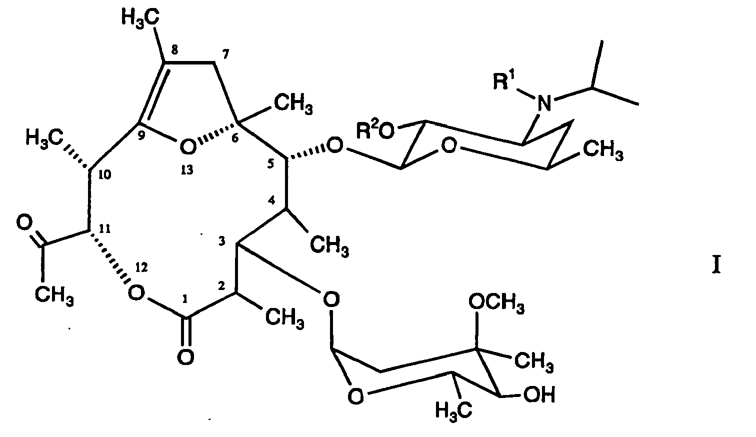 worin
R¹ Wasserstoff oder Methyl bedeutet und
R² Wasserstoff oder C₁₋₄-Alkylcarbonyl bedeutet
und deren stabilen und physiologisch verträglichen Säureadditionssalzen, **dadurch gekennzeichnet, daß** man in einer Vierbindung der allgemeinen Formel II, 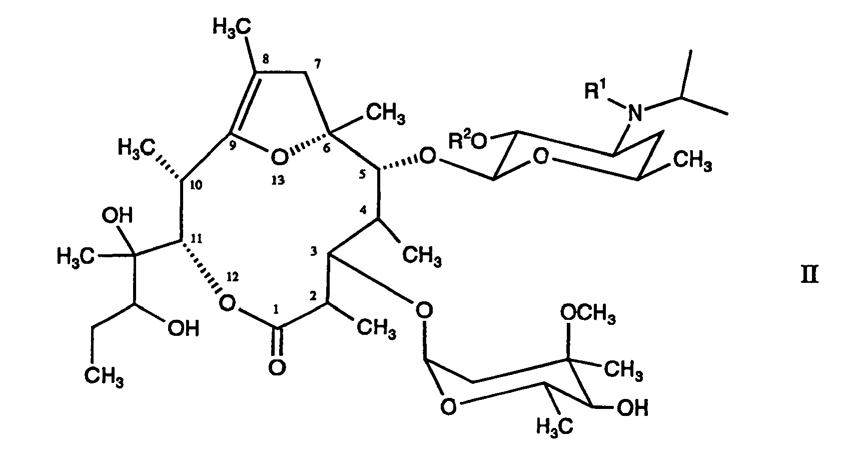 worin R¹ und R² obige Bedeutungen besitzen, die 2',3'-Dihydroxypent-2'-yl-Seitenkette in 11-Position des cyclischen Grundgerüstes durch oxidative Glykolspaltung in eine Acetyl-Seitenkette überführt.

6. Verfahren nach Anspruch 5, worin man in die erhaltene Verbindung der Formel I, worin R¹ Wasserstoff bedeutet, einen Methylrest R¹ einführt oder in der erhaltenen Verbindung der Formel I, worin R¹ Methyl bedeutet, den Methylrest R¹ abspaltet.

7. Verfahren nach Anspruch 5 oder 6, worin man freie Verbindungen der Formel I in ihre stabilen und physiologisch verträglichen Säureadditionssalze überführt oder die Säureadditionssalze in die freien Verbindungen der Formel I überführt.

## Claims

1. (2R,3S,4S,5R,6R,10R,11R)-2,4,6,8,10-pentamethyl-11-acetyl-12,13-dioxabicyclo[8.2.1]tridec-8-en-1-one compounds of the general formula I 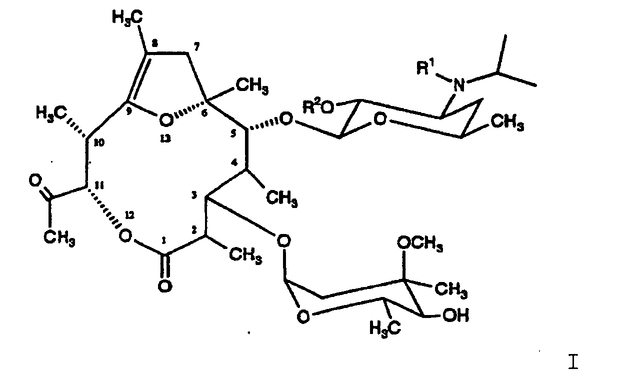 in which
R¹ is hydrogen or methyl and
R² is hydrogen or C₁₋₄-alkylcarbonyl
and stable and physiologically compatible acid addition salts thereof.

2. Compounds according to Claim 1, in which R¹ is methyl.

3. Compounds according to one of the preceding claims, in which R² is hydrogen.

4. Pharmaceutical composition comprising a pharmacologically active amount of a compound according to Claim 1 and customary pharmaceutical auxiliaries and/or excipients.

5. Process for the preparation of (2R,3S,4S,5R,6R,10R,11R)-2,4,6,8,10-pentamethyl-11-acetyl-12,13-dioxabicyclo[8.2.1]tridec-8-en-1-one compounds of the general formula I 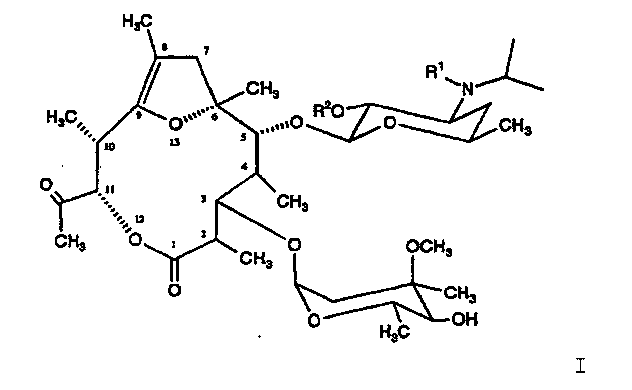 in which
R¹ is hydrogen or methyl and
R² is hydrogen or C₁₋₄-alkylcarbonyl
and stable and physiologically compatible acid addition salts thereof, **characterized in that**, in a compound of the general formula II, 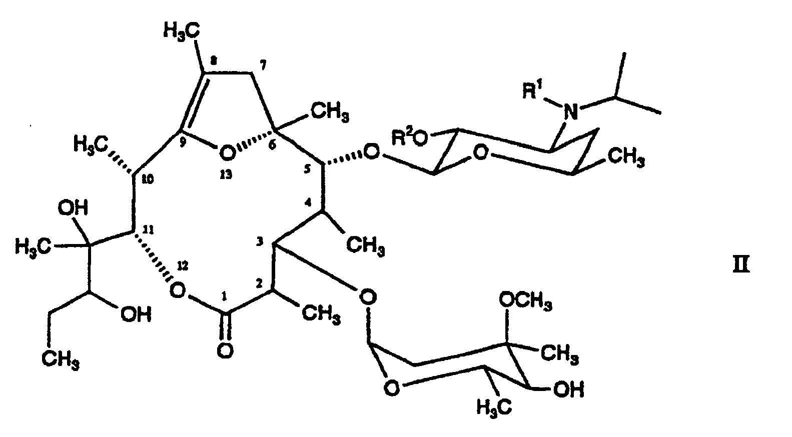 in which R¹ and R² have the above meanings, the 2', 3'-dihydroxypent-2'-yl side chain in the 11-position of the cyclic basic backbone is converted to an acetyl side chain by means of oxidative glycol cleavage.

6. Process according to Claim 5, in which, into the resulting compound of the formula I in which R¹ is hydrogen, a methyl radical R¹ is introduced, or, in the resulting compound of the formula I in which R¹ is methyl, the methyl radical R¹ is cleaved off.

7. Process according to Claim 5 or 6, in which free compounds of the formula I are converted to their stable and physiologically compatible acid addition salts or the acid addition salts are converted to the free compounds of the formula I.

## Revendications

1. Composés (2R,3S,4S,5R,6R,10R,11R)-2,4,6,8,10-pentaméthyl-11-acétyl-12,13-dioxabicyclo[8.2.1]tridéc-8-én-1-one de formule générale I 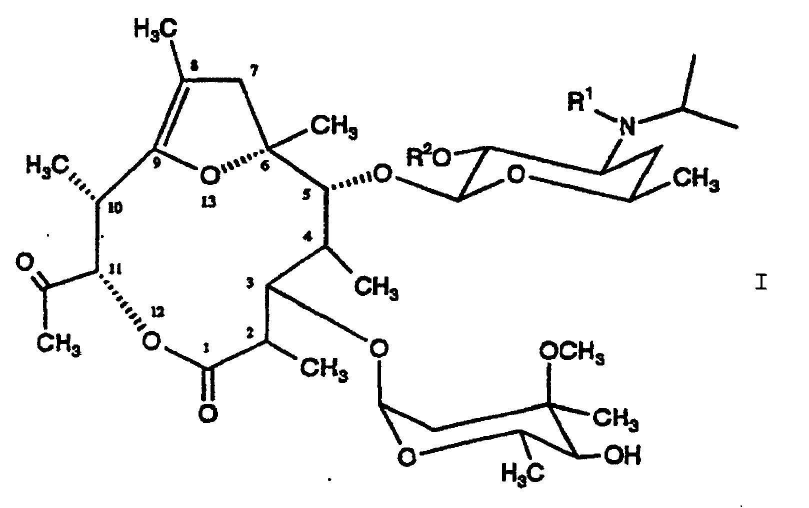 dans laquelle
R¹ représente un atome d'hydrogène ou le groupe méthyle et
R² représente un atome d'hydrogène ou un groupe alkyl(C₁-C₄)carbonyle
et leurs sels d'addition avec des acides, stables et physiologiquement acceptables.

2. Composés selon la revendication 1, dans lesquels R¹ représente le groupe méthyle.

3. Composés selon l'une quelconque des revendications précédentes, dans lesquels R² représente un atome d'hydrogène.

4. Médicament, contenant une quantité pharmacologiquement efficace d'un composé selon la revendication 1 et des adjuvants et/ou véhicules pharmaceutiques usuels.

5. Procédé pour la préparation de composés (2R,3S,4S,5R,6R,10R,11R)-2,4,6,8,10-pentaméthyl-11-acétyl-12,13-dioxabicyclo[8.2.1]tridéc-8-én-1-one de formule générale I 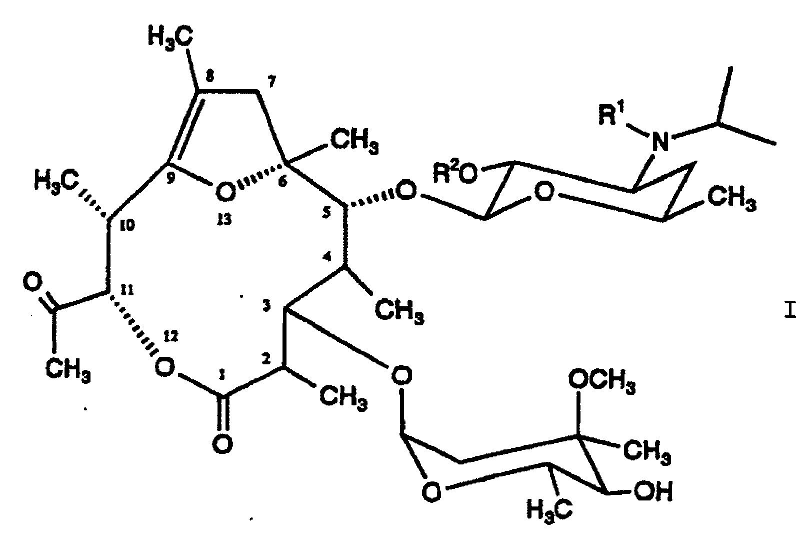 dans laquelle
R¹ représente un atome d'hydrogène ou le groupe méthyle et
R² représente un atome d'hydrogène ou un groupe alkyl (C₁-C₄) carbonyle
et de leurs sels d'addition avec des acides, stables et physiologiquement acceptables, **caractérisé en ce que** dans un composé de formule II, 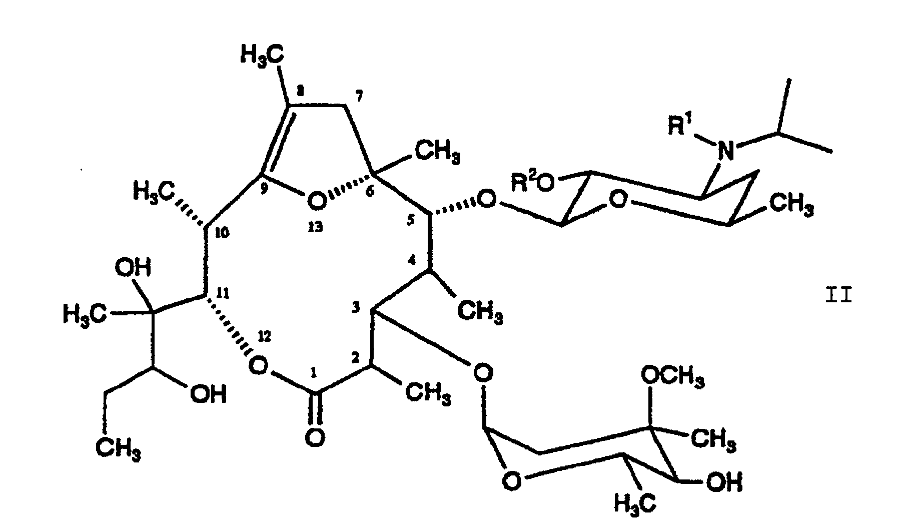 dans laquelle R¹ et R² ont les significations ci-dessus, on convertit la chaîne latérale 2',3'-dihydroxypent-2'-yle, en position 11 de la structure cyclique de base, en une chaîne latérale acétyle par élimination de glycol par oxydation.

6. Procédé selon la revendication 5, dans lequel on introduit un radical méthyle R¹ dans le composé de formule I obtenu, dans lequel R¹ représente un atome d'hydrogène, ou on élimine le radical méthyle R¹ du composé de formule I obtenu, dans lequel R¹ représente le groupe méthyle.

7. Procédé selon la revendication 5 ou 6, dans lequel on convertit des composés libres de formule I en leurs sels d'addition avec des acides, stables et physiologiquement acceptables ou on convertit les sels d'addition en les composés de formule I libres.
